# EUROPEAN PATENT SPECIFICATION

(11) **EP 2 968 960 B1**
(45) Date of publication and mention of the grant of the patent: **13.09.2017**
(21) Application number: 14708709.2
(22) Date of filing: 12.02.2014
(51) Int. Cl.: A61N 1/378, A61M 5/142, A61N 1/37, G01R 31/36, A61N 1/372

(54) **GRAPHICAL DISPLAY OF REMAINING LONGEVITY OF ENERGY SOURCE OF IMPLANTABLE MEDICAL DEVICE**
GRAFISCHE ANZEIGE DER VERBLEIBENDEN LEBENSDAUER DER ENERGIEQUELLE EINER IMPLANTIERBAREN MEDIZINISCHEN VORRICHTUNG
DISPOSITIF D'AFFICHAGE GRAPHIQUE DE LONGÉVITÉ RESTANTE DE SOURCE D'ÉNERGIE DE DISPOSITIF MÉDICAL IMPLANTABLE

(30) Priority: 15.03.2013 US 201313835905
(43) Date of publication of application: 20.01.2016
(73) Proprietor: Medtronic, Inc., Minneapolis, MN 55432 (US)
(72) Inventor: YOUNKER, Gregory, A., Minneapolis, MN 55432 (US); UKO, Idara, D., Minneapolis, MN 55432 (US)
(74) Representative: Hughes, Andrea Michelle
(86) International application number: PCT/US2014/015936
(87) International publication number: WO 2014/149252

(56) References cited:
- WO-A1-2009/078905
- US-A1- 2012 265 266
- US-B1- 7 469 161

## Description

### FIELD

The present description relates generally to power source monitors and, more particularly, to power source monitors for implantable medical devices having an energy source having a voltage which declines over its useful life. The invention is set out in the appended claims.

### BACKGROUND

The medical device industry produces a wide variety of electronic and mechanical devices for treating patient medical conditions. Depending upon the medical condition, medical devices can be surgically implanted or connected externally to the patient receiving treatment. Clinicians use medical devices alone or in combination with therapeutic substance therapies and surgery to treat patient medical conditions. For some medical conditions, medical devices provide the best, and sometimes the only, therapy to restore an individual to a more healthful condition and a fuller life.

One type of medical device is an implantable therapeutic substance infusion device. An implantable therapeutic substance infusion device is implanted by a clinician into a patient at a location appropriate for the therapy. Typically, a therapeutic substance infusion catheter is connected to the device outlet and implanted to infuse the therapeutic substance such as a drug or infusate at a programmed infusion rate and predetermined location to treat a condition such as pain, spasticity, cancer, and other medical conditions. Many therapeutic substance infusion devices are configured, so the device can be replenished with therapeutic substance through a septum while the device is implanted, so the time the device can be implanted may not be limited by therapeutic substance capacity. An example of an implantable therapeutic substance infusion is shown in Medtronic, Inc. product brochure entitled "SynchroMed™ Infusion System" (1995).

Other implantable devices exist which electrically stimulate neurological tissue to treat or relieve the symptoms of a wide variety of physiological or psychological maladies or pain. Such devices are typically part of systems that are entirely implantable within the patient or are partially implantable and partially external to the patient. Systems that are entirely implantable in the patient typically include an implantable pulse generator and an extension and lead or leads. In such a system, the implantable pulse generator, extension and lead are entirely implanted in the bodies of the patients. An example of such a system is the Itrel™ 3 system manufactured and sold by Medtronic, Inc. of Minneapolis, Minnesota. Because the implantable pulse generator is implanted, the power sources needed to power the implantable pulse generator are also implanted. Typically, the power source for an implantable pulse generator is a battery.

Each of these implantable devices delivers a therapeutic output to the patient. In the case of an implantable therapeutic substance infusion device, the therapeutic output can be a therapeutic substance which is infused into the patient. In the case of a neurological tissue stimulator, the therapeutic output is an electrical signal intended to produce a therapeutic result in the patient. Other types of implantable therapeutic delivery devices also exist including cardiac pacemakers and defibrillators.

Electrically powered implanted therapeutic delivery devices can require replacement once implanted due to factors such as battery consumption, corrosive damage and mechanical wear. Since replacement of the implanted therapeutic delivery device requires an invasive procedure of explanting the existing device and implanting a new device, it is desirable to only replace the therapeutic delivery device when replacement is required. Replacement of previously implanted therapeutic delivery devices was typically scheduled based upon a worst-case statistically forecasted elective replacement period. The worst-case scenario typically resulted in the implanted therapeutic delivery device being replaced several months or even years before the implanted therapeutic delivery device actually required replacement.

U.S. Patent No. 6,901,293, Rogers et al, discloses a power source longevity monitor for an implantable medical device. The monitor uses an energy counter that counts the amount of energy used by the implantable medical device. An energy converter converts the energy used into an estimate of remaining power source longevity and generates an energy longevity estimate. A voltage monitor monitors the voltage of the power source. A voltage converter converts the voltage monitored by the voltage monitor into an estimate of remaining longevity of the power source and generates a voltage longevity estimate. A calculator predicts the power source longevity using the energy longevity estimate early in the useful life of the power source and uses the voltage longevity estimate later in the useful life of the power source. WO-A-2009/078905 and US-A-7,469,161 disclose different devices operable to calculate and graphically display power consumption of an implantable medical device. US-A-2012/0265266 disclose a system for estimating longevity of an implantable medical device.

### SUMMARY

Thus, there have been devices and methods for determining, estimating and/or calculating a state of charge or discharge of an energy source of an implantable medical device. Determining, estimating and/or calculating the state of charge or discharge of the energy source is important information for the patient and for the clinician.

An energy source in an implantable medical device has several distinct states of discharge. A first stage of depth of discharge can be correlated to the beginning of service. A second stage of depth of discharge can be correlated to a recommended replacement time. A third stage of depth of discharge can be correlated to end of service.

Presenting the charge or discharge information, particularly as to how the state of discharge relates to these distinct states of discharge in an intuitive and meaningful way, in a way that is easy for the patient and/or the clinician to recognize, understand and not misunderstand can significantly aid in quickly and unmistakably determining the current state of discharge of the energy source.

Displaying the information in a scaled, graphical format makes the information easily understandable. Adding different colors to different distinct stages makes it even further easier to see and understand.

In an embodiment, an implantable medical device has an energy source having a longevity characterized by a depth of discharge representative of a first stage of discharge, a second stage of discharge and a third stage of discharge. Electrical circuitry is configured to calculate a remaining longevity of the energy source. A display is configured to display in graphical form using a scale length representative of a time between the first stage of discharge and the third stage of discharge. A first portion of the display between the second stage of discharge and the third stage of discharge is indicated in a first color. A second portion of the display between the remaining longevity and the second stage of discharge is indicated in a second color. A third portion of the display between the first stage of discharge and the remaining longevity is indicated in a third color.

In an embodiment, the first stage of discharge is representative of beginning of service, the second stage of discharge is representative of recommended replacement time and the third stage of discharge is representative of end of service.

In an embodiment, the graphical form is a bar graph.

In an embodiment, the energy source is a battery.

In an embodiment, the electrical circuitry is configured to calculate the remaining longevity using at least one of a voltage of the battery and a count of energy consumed and delivered by the implantable medical device.

In an embodiment, the electrical circuitry is configured to calculate the remaining longevity using the voltage of the battery.

In an embodiment, the electrical circuitry is configured to calculate the remaining longevity using the count of energy consumed and delivered by the implantable medical device.

In an embodiment, the electrical circuitry is configured to calculate the remaining longevity using a combination of the voltage of the battery and the count of energy consumed and delivered by the implantable medical device.

In an embodiment, an implantable medical device capable of being configured to deliver a plurality of electrical activities has an energy source, e.g., a battery, having a longevity. Electrical circuitry is configured to calculate a remaining longevity of the energy source separately for each of the plurality of electrical activities. The energy source has a predetermined expected longevity for each of the plurality of electrical activities. A communication medium, e.g., display, is configured to display, for each of the plurality of electrical activities, in graphical form an indication representative of the remaining longevity compared with the expected longevity for each respective one of the plurality of electrical activities.

In an embodiment, the graphical form comprises a scale having a length representative of time with the scale showing indicia of time meeting the predetermined expected longevity and the remaining longevity is displayed, separately for each of the plurality of electrical activities, along the scale in relation to the indicia of time meeting the predetermined expected longevity for each respective one of the plurality of electrical activities.

In an embodiment, the scale shows indicia of time below the predetermined expected longevity and shows indicia of time exceeding the predetermined expected longevity for each respective one of the plurality of electrical activities.

In an embodiment, the graphical form provides a common view with an indication representative of the remaining longevity compared with the expected longevity for all of the plurality of electrical activities so that remaining longevity compared with the predetermined expected longevity can be compared among all of the plurality of electrical activities.

In an embodiment, the implantable medical device is a heart stimulator and the plurality of electrical activities are at least two of atrial pacing, right ventricular pacing, left ventricular pacing, telemetry and charging.

In an embodiment, a device-implemented method displays a longevity of an energy source of an implantable medical device, the longevity of the energy source being characterized by a depth of discharge representative of a first stage of discharge, a second stage of discharge and a third stage of discharge. The implantable medical device calculates a remaining longevity (RL) of the energy source. A time between the first stage of discharge and the third stage of discharge is displayed in graphical form using a scale length representative of time. A first portion of the display between the second stage of discharge and the third stage of discharge is indicated in a first color. A second portion of the display between the remaining longevity and the second stage of discharge is indicated in a second color. A third portion of the display between the first stage of discharge and the remaining longevity being indicated in a third color.

In an embodiment, the first stage of discharge is representative of beginning of service, the second stage of discharge is representative of recommended replacement time and the third stage of discharge is representative of end of service.

In an embodiment, the remaining longevity is calculated using at least one of a voltage of the battery and a count of energy consumed and delivered by the implantable medical device.

In an embodiment, the remaining longevity is calculated using the voltage of the battery.

In an embodiment, the remaining longevity is calculated using the count of energy consumed and delivered by the implantable medical device.

In an embodiment, the remaining longevity is calculated using a combination of the voltage of the battery and the count of energy consumed and delivered by the implantable medical device.

In an embodiment, the implantable medical device is capable of being configured to deliver a plurality of electrical activities and wherein the energy source has a predetermined expected longevity for each of the plurality of electrical activities, the calculating step is accomplished by calculating a remaining longevity of the energy source separately for each of the plurality of electrical activities and the displaying step is accomplished by displaying, for each of the plurality of electrical activities, in graphical form an indication representative of the remaining longevity compared with the expected longevity.

In an embodiment, the graphical form comprises a scale length representative of time with the scale showing indicia of time meeting the predetermined expected longevity, and the displaying step is accomplished by displaying the remaining longevity, separately for each of the plurality of electrical activities, along the scale in relation to the indicia of time meeting the predetermined expected longevity.

In an embodiment, the graphical form is a scale showing indicia of time below the predetermined expected longevity and showing indicia of time exceeding the predetermined expected longevity.

In an embodiment, the graphical form provides a common view with an indication representative of the remaining longevity compared with the expected longevity for all of the plurality of electrical activities so that remaining longevity compared with the predetermined expected longevity can be compared among all of the plurality of electrical activities.

In an embodiment, the implantable medical device comprises a heart stimulator and the plurality of electrical activities comprises at least two of atrial pacing, right ventricular pacing, left ventricular pacing, telemetry and charging.

In an embodiment, a device-implemented method displays a longevity of an energy source of an implantable medical device capable of being configured to deliver a plurality of electrical activities. The implantable medical device calculates a remaining longevity of the energy source for each of the plurality of electrical activities. An indication representative of the remaining longevity, for each of the plurality of electrical activities, compared with an expected longevity for each respective one of the plurality of electrical activities is displayed.

In an embodiment, the graphical form is a scale having a length representative of time with the scale showing indicia of time meeting the predetermined expected longevity, and the displaying step is accomplished by displaying the remaining longevity, separately for each of the plurality of electrical activities, along the scale in relation to the indicia of time meeting the predetermined expected longevity for each respective one of the plurality of electrical activities.

In an embodiment, the scale shows indicia of time below the predetermined expected longevity and shows indicia of time exceeding the predetermined expected longevity for each respective one of the plurality of electrical activities.

In an embodiment, the displaying step provides a common view with an indication representative of the remaining longevity compared with the expected longevity for all of the plurality of electrical activities so that remaining longevity compared with the predetermined expected longevity can be compared among all of the plurality of electrical activities. The present invention is set out in the appended claims. The embodiments, aspects or examples according to the present description that do not fall within the scope of said claims are provided for illustrative purposes only and do not form part of the present invention.

### FIGURES

**Figure 1** is a block diagram of a medical system employing a graphical energy source longevity display for an implantable medical device;
**Figure 2** is a graph illustrating distinct states of discharge of an energy source for an implantable medical device;
**Figure 3** is a display with a graphical scale illustrating the longevity of the energy source;
**Figure 4** is a display with a graphical scale illustrating the longevity of the energy source at pre-implant stage;
**Figure 5** is an alternative display with a graphical scale illustrating the longevity of the energy source at pre-implant stage;
**Figure 6** is a display with a graphical scale illustrating the longevity of the energy source with approximately 3.3 years of useful life remaining;
**Figure 7** is an alternative display illustrating the longevity of the energy source at recommended replacement time; and
**Figure 8** is a flow chart which illustrates displaying a graphical scale illustrating the longevity of the energy source.

### DESCRIPTION

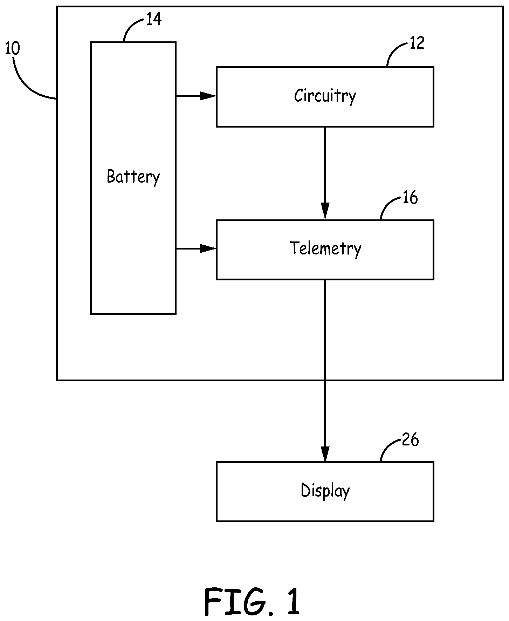

**Figure 1** is a simplified block diagram of implantable medical device 10 containing circuitry 12 which generically performs control and therapy delivery functions. Therapy delivery may be electrical stimulation, e.g., a heart stimulator such as a pacemaker, defibrillator or combination device, a drug delivery device, e.g., a drug pump, or other implantable medical device having electrically powered circuitry 12. An energy source, e.g., battery 14, supplies power to circuitry 12 for device operation and therapy delivery, if appropriate. Implantable medical device 10 may communicate with the outside world while implanted using telemetry unit 16 which is also powered by battery 14. Generic construction and generic operation of circuitry 12, battery 14 and telemetry unit 16 is conventional and well known in the industry, for example, as exemplified by the device and operation described in U.S. Patent No. 6,901,293.

Circuitry 12 uses conventional techniques to determine, estimate and/or calculate the state of discharge of battery 14. Battery 14 has a distinct state of discharge when initially implanted representative of being fully charged or nearly fully charge referred to as beginning of service (BOS). Implantable medical device then operates for a considerable period over its service life providing therapeutic delivery. In typical implantable devices, the length of service may be in the range of five (5) to seven (7) years. As the depth of discharge of battery 14 reaches or nears a point in time at which the battery should be replaced or, in an embodiment, recharged, the depth of discharge of battery 14 reaches a distinct state referred to as a recommended replacement time (RRT). As battery 14 continues to discharge, if not replaced or recharged, battery 14 will eventually reach a distinct state of discharge in which battery 14 is no longer serviceable, i.e., can no longer consistently and safely operate implantable medical device 10 referred to as a end of service (EOS).

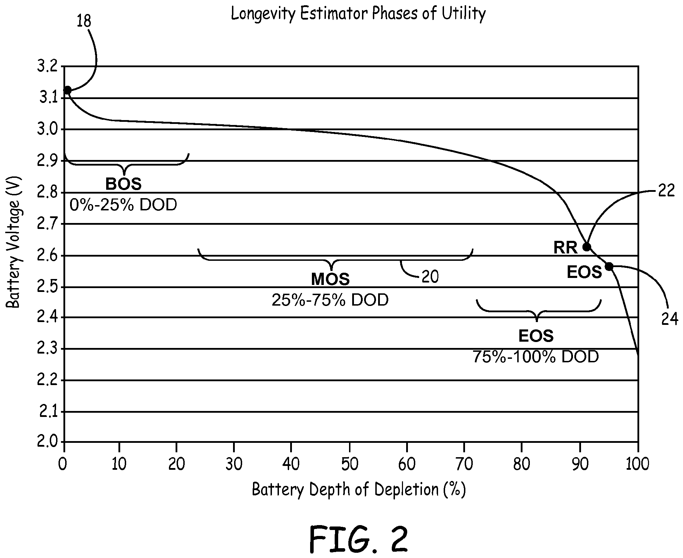

**Figure 2** is a graph illustrating the voltage of a typical battery 14 used in an implantable medical device 10 showing the battery voltage (vertical axis) versus the depth of discharge (horizontal axis). As the depth of discharge increases as the battery is used, the horizontal axis also is roughly representative of time of use. Battery 14 has its maximum voltage of just over 3.1 Volts at zero depth of discharge at discharge stage "beginning of service" 18. After declining noticeably in the first few percent of depth of discharge, the voltage of battery 14 becomes much more stable over the main useful life stage identified as "middle of service" 20. As battery 14 gets closer to the end of its usefulness in implantable medical device 10 the voltage of battery 14 begins to decline more sharply. Before reaching the end of service point in the depth of discharge curve, battery 14 reaches a distinct stage of depth of discharge where battery 14 should be replaced allowing a safety margin for continued operation at least until that point, represented on the curve as 'recommended replacement time" 22. This is typically the point by which the patient will return to the clinician for device 10 or battery 14 replacement or, in an embodiment, recharging. If battery 14 is allowed to continue to discharge, "end of service" 24 discharge stage will be reached where battery 14 may no longer be reliably serviceable.

It is important, of course, for the patient or the clinician or both the patient and clinician to know the state of discharge of battery 14. It is important that it be known when recommended replacement time 22 is reached so the patient and the clinician may make arrangements for battery 14 to be replaced or recharged. And, if recommended replacement time 22 is passed on the depth of discharge curve, it is also important to know when end of service 24 is reached.

It is also important to know not only when recommended replacement time 22 and/or end of service 24 is reached but also how much time or how much usage of battery 14 is available to the patient before recommended replacement time 22 and end of service 24 is reached. Knowing how much time or how much usage can give the patient and/or the clinician confidence and comfort that the patient may continue to use implantable medical device 10. In this regard, it also may be useful to know how far away from beginning of service 18 battery 14 is on the depth of discharge curve.

Implantable medical device 10 is configured to communicate the state of depth of discharge from implantable medical device 10 to an external device or medium for reading and interpretation by the patient and/or clinician. Telemetry unit 16 of implantable medical device 10 **(****Figure 1****)** may communicate depth of discharge information to a patient programmer or control unit or physicians programmer external to the patient. Telemetry is done through well known conventional techniques.

Communicating the stage of discharge of battery 14 in numerical form is useful but is not as immediately recognizable and intuitive as a graphical display of the state of discharge. Display 26 which may be located in or on a patient programmer or control unit, a physician or clinician programmer or other visual communication medium, such as a local or remote desktop, laptop or mobile device. Display 26 may provide depth of discharge information of battery 14 in a graphical, scalable format for each recognition of the actual state of the depth of discharge of battery 14 including how the current depth of discharge relates to know distinct stages of discharge, such as beginning of service 18, recommended replacement time 22 and end of service 24.

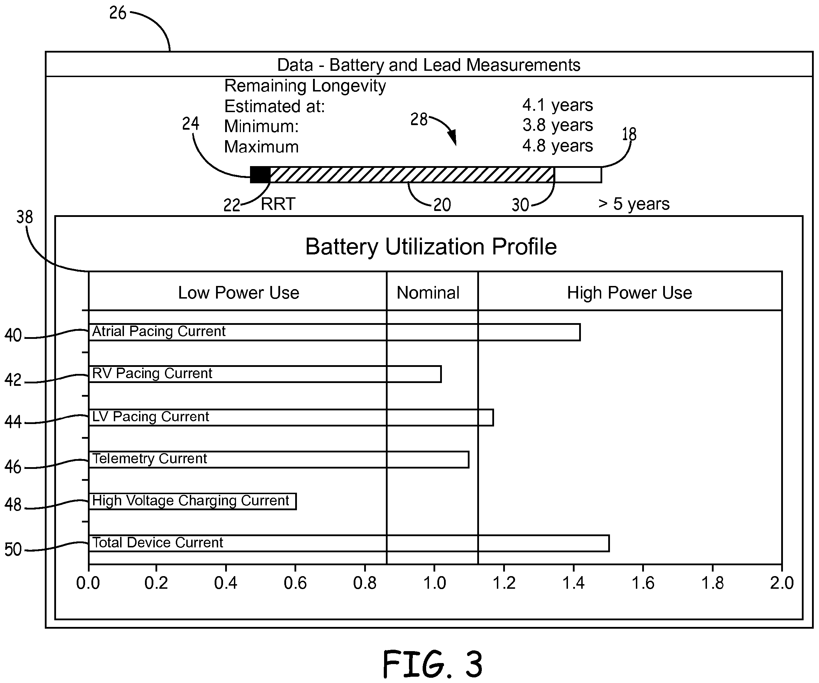

**Figure 3** provides an illustration of a graphical display 26 having graphical scale 28 having a length representative of a time between beginning of service 18 and end of service 24. The current state 30 of the depth of discharge of battery 14 in scale 28 is near beginning of service 18 representing over five (5) years of useable battery life remaining. Scale 28 is color coded. The portion of scale 28 between the current state 30 of depth of discharge of battery 14 to recommended replacement time, essentially the remaining useful life of battery 14, is displayed in a first color, namely green to indicate that the situation is under control. The portion of scale 28 between recommended replacement 22 and end of service 24 is displayed in a second color, namely red to indicate the severity of the situation. The portion of scale 28 between the current state 30 of depth of discharge and beginning of service, essentially the portion of the battery life already consumed, is displayed in a third color, namely white indicative of past usage. It is to be recognized and understood, of course, that different colors may be used for each portion or segment of scale 28.

Illustrating the depth of discharge of battery 14 with scale 28 as illustrated in **Figure 3** provides an easy and well understood means of easily communicating the depth of discharge of battery 14 and which makes it easy for the viewer to comprehend the relative status of battery 14 and the relative time periods available before battery 14 should be replaced or recharged.

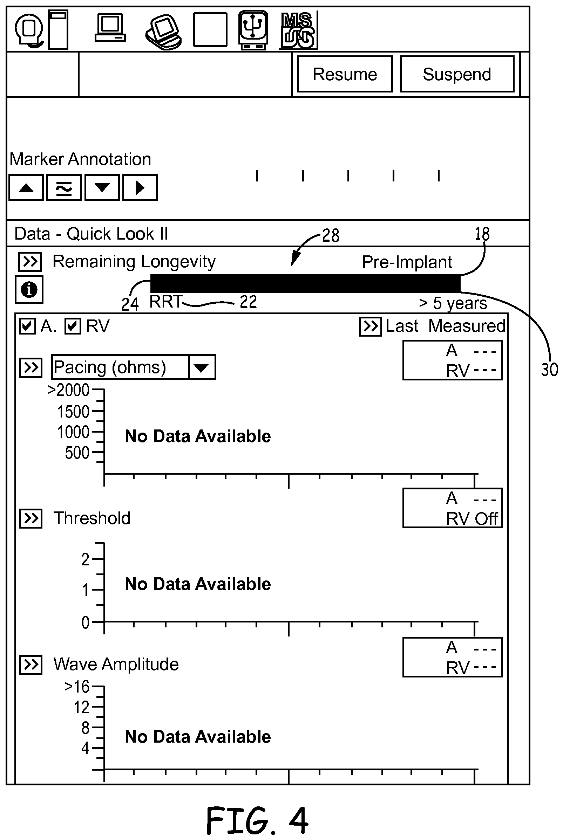

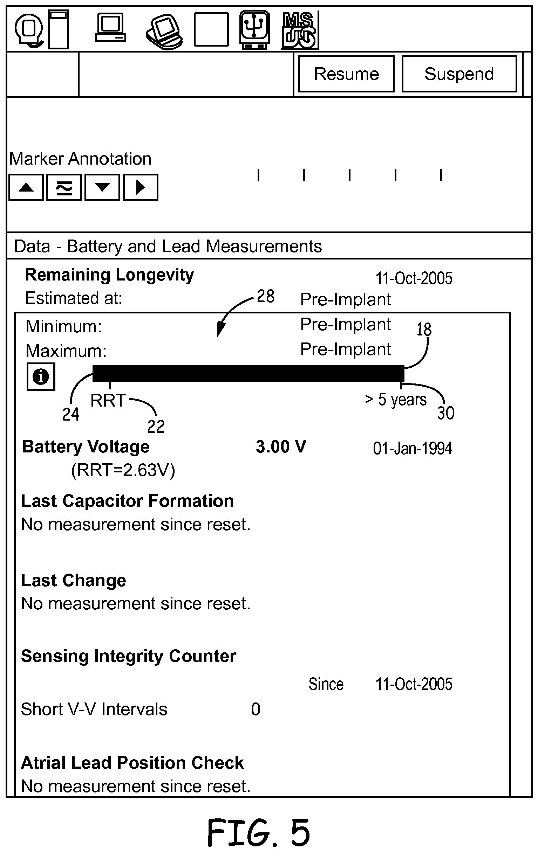

**Figure 4** and **Figure 5** illustrate graphical displays of the depth of discharge of battery 14 as part of a display also illustrating other aspects of implantable medical device 10. Scale 28 is illustrated as in **Figure 3** with a red portion (shown in coloured black here) between end of service 24 and recommended replacement time 22, a green portion (shown by hashed lines) between recommended replacement time 22 and the current state 30 of depth of discharge. Since **Figure 4** is illustrative of battery 14 in pre-implant stage, battery 14 is fully charged so that the current state 30 of depth of discharge is identical or nearly identical to beginning of service 18 and, hence, there is no white portion of scale 28. Again, scale 28 provides an easy and well understood means of easily communicating the depth of discharge of battery 14 and which makes it easy for the viewer to comprehend the relative status of battery 14 and the relative time periods available before battery 14 should be replaced or recharged.

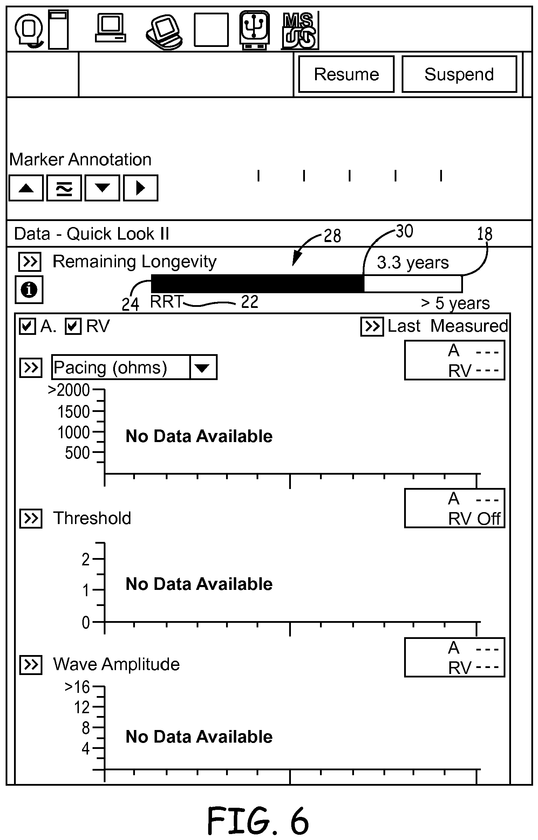

**Figure 6** illustrates a graphical display of depth of discharge of battery 14 in the useful life of battery 14 with a current state 30 of depth of discharge of about 3.3 years of useful life remaining. Again, the portion of scale 28 between the current state 30 of depth of discharge of battery 14 to recommended replacement time 22, essentially the remaining useful life of battery 14, is displayed in green. The portion of scale 28 between recommended replacement 22 and end of service 24 is displayed in red (coloured black here). The portion of scale 28 between the current state 30 of depth of discharge and beginning of service 18, essentially the portion of the battery life already consumed, is displayed in white. This provides an easy and well understood means of easily communicating the depth of discharge of battery 14 and which makes it easy for the viewer to comprehend the relative status of battery 14 and the relative time periods available before battery 14 should be replaced or recharged.

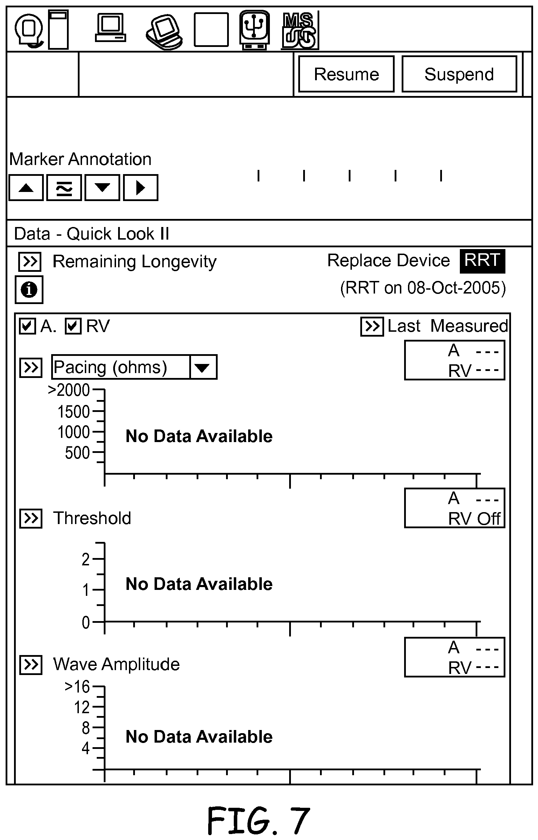

**Figure 7** illustrates the display with the current state 30 of depth of discharge of battery 14 reaching recommended replacement time 22. Although scale 28 may be continued to be displayed in this situation, in an embodiment, scale 28 is replaced with a notation "Replace Device" along with a red (shown black here) indication "RRT" indicating that recommended replacement time has been reached.

In an embodiment, **Figure 3** provides graphical display 38 of determined, estimated and/or calculated longevity for a plurality of electrical activities, namely atrial pacing 40, right ventricular pacing 42, left ventricular pacing 44, telemetry 46, high voltage charging current 48 and total device current 50. Graphical display 38 displays, separately for each distinct electrical activity, a bar representative of a longevity of battery 14 should implantable medical device be operated to perform each respective electrical activity. The horizontal axis is representative of time of longevity, normalized with normal or expected longevity being represented as 1.0. Graphical display 38 is also broken into separate sections. A center section, from a normalized 0.85 to 1.15, is indicative of a battery longevity which generally meets the expected battery longevity profile. The center section is colored, or has a header colored, white. A left section, from a normalized 0.0 to .085, is indicative of low power use which would result in a battery longevity which is greater than expected battery longevity. The left section is colored, or has a header colored, green (colours not shown in the drawings). A right section, from a normalized 1.15 to 2.0, is indicative of high power use which would result in a lower than expected battery longevity. The right section is colored, or has a header colored, red (colours not shown in the drawings). It is to be recognized and understood that the horizontal axis is generally representative of an expectation, in time, of how the calculated battery longevity compares with an originally expected battery longevity. Although the horizontal axis of graphical display 38 is shown normalized that other displays are possible including a direct indication of time, for example in years of expected life. If the horizontal axis was representative of time, then a longer bar (to the right) would be indicative of greater than expected longevity and a shorter bar (to the left) would be indicative of lower than expected longevity.

Atrial pacing activity 40, having a normalized battery longevity of around 1.45, has a bar which graphically extends into the right section of graphical display 38 indicative of high power use and a lower than expected longevity.

Right ventricular pacing activity 42, having a normalized battery longevity of around 1.0, has a bar which graphically extends into the center section of graphical display 38 indicative of meeting expected longevity.

Left ventricular pacing activity 44, having a normalized battery longevity of around 1.2, has a bar which graphically extends slightly into the right section of graphical display 38 indicative of being slightly high power use and being slightly below expected or published expected longevity.

Telemetry current 46, having a normalized battery longevity of just under 1.1, has a bar which graphically extends into the far right hand portion of the center section of graphical display 38 indicative of meeting expected longevity.

High voltage charging current activity 48, having a normalized battery longevity of around 0.6, has a bar which graphically extends only into the left section of graphical display 38 indicative of low power use and a higher than expected longevity.

Total device current 50, having a normalized battery longevity of around 1.5, has a bar which graphically extends into the right section of graphical display 38 indicative of high power use and a lower than published or expected longevity.

By graphically displaying battery longevity calculated for each of the plurality of electrical activities (40, 42, 44, 46, 48 and 50) together in graphical display 38, the user, typically the patient and/or the clinician, can easily visually see not only whether the implantable medical device is calculated to meet or exceed expected or published longevity or not. Further, the user can easily compare and understand how the differing electrical activities affect the longevity of battery 14. For example, graphical display 38 clearly indicates that both atrial pacing activity 40 and high voltage charging current activity 48 are high power uses and will result in decreased battery longevity. Conversely, high voltage charging current activity 48 is a low power use and will result in increased battery longevity. By easily and graphically comparing battery longevities for each separate electrical activity together on a single graphical display 38, the clinician, for example, can easily recognize that if implantable medical device 10 is operated in left ventricular pacing activity 44, that battery longevity will be severely compromised. Having this information, the clinician can then make a decision as to whether to utilize left ventricular pacing or another form of less battery intrusive activity or how much of a particular electrical activity to utilize.

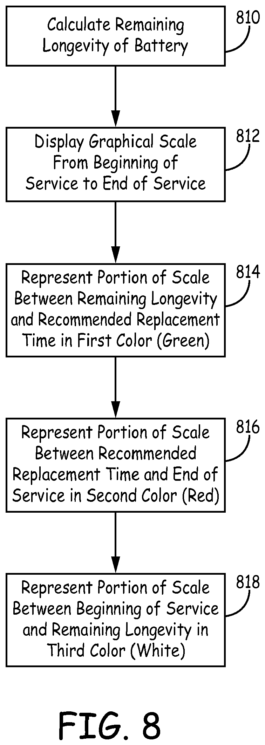

In **Figure 8****,** the remaining longevity of battery 14 is calculated (810) according to well known, conventional techniques. The remaining longevity of battery 14 is the equivalent of the current state 30 of the depth of discharge of battery 14. The current state 30 of the depth of discharge of battery 14 is displayed (812) in graphical scale 28 having a length from beginning of service 18 to end of service 24. A first portion of scale 28 between the current state 30 of depth of discharge of battery 14 to recommended replacement time 22, essentially the remaining useful life of battery 14, is displayed (814) in a first color, such as green. A second portion of scale 28 between recommended replacement time 22 and end of service 24 is displayed (816) in a second color, such as red. A third portion of scale 28 between the current state 30 of depth of discharge and beginning of service 18, essentially the portion of the battery life already consumed, is displayed (818) in a third color, such as white.

## Claims

1. A medical system, comprising:
an implantable medical device (10) having:
an energy source (14) operatively coupled to and powering said electrical circuitry, said energy source having a longevity **characterized by** a depth of discharge representative of a first stage of discharge, a second stage of discharge and a third stage of discharge; and
electrical circuitry (12) operatively coupled to and being powered by said energy source, said electrical circuitry being configured to calculate a remaining longevity of said energy source; and
a display (26), operatively coupled to said electrical circuitry, configured to display in graphical form using a scale length representative of a time between said first stage of discharge and said third stage of discharge;
a first portion of said display between said second stage of discharge and said third stage of discharge being indicated in a first color;
a second portion of said display between said remaining longevity and said second stage of discharge being indicated in a second color;
a third portion of said display between said first stage of discharge and said remaining longevity being indicated in a third color; and
wherein said implantable medical device (10) is capable of being configured to deliver a plurality of electrical activities;
wherein said electrical circuitry (12) is further configured to calculate a remaining longevity of said energy source separately for each of said plurality of electrical activities;
wherein said energy source (14) has a predetermined expected longevity for each of said plurality of electrical activities;
wherein said display (26) is configured to display, for each of said plurality of electrical activities, in graphical form an indication representative of said remaining longevity compared with said expected longevity.

2. The medical system of claim 1:
wherein said first stage of discharge is representative of beginning of service;
wherein said second stage of discharge is representative of recommended replacement time; and
wherein said third stage of discharge is representative of end of service.

3. The medical system of claim 1 or 2 wherein said graphical form comprises a bar graph.

4. The medical system of any preceding claim wherein said energy source comprises a battery.

5. The medical system of any preceding claim wherein said electrical circuitry is configured to calculate said remaining longevity using at least one of a voltage of said battery and a count of energy consumed and delivered by said implantable medical device.

6. The medical system of claim 5 wherein said electrical circuitry is configured to calculate said remaining longevity using said voltage of said battery.

7. The medical system of claim 5 wherein said electrical circuitry is configured to calculate said remaining longevity using said count of energy consumed and delivered by said implantable medical device.

8. The medical system of claim 5 wherein said electrical circuitry is configured to calculate said remaining longevity using a combination of said voltage of said battery and said count of energy consumed and delivered by said implantable medical device.

9. The medical system of claim 1:
wherein said graphical form comprises a scale length representative of time with said scale showing indicia of time meeting said predetermined expected longevity; and
wherein said remaining longevity is displayed, separately for each of said plurality of electrical activities, along said scale in relation to said indicia of time meeting said predetermined expected longevity.

10. The medical system of claim 9:
wherein said graphical form further comprises said scale showing indicia of time below said predetermined expected longevity and showing indicia of time exceeding said predetermined expected longevity.

11. The medical system of claim 9 or 10 wherein said graphical form provides a common view with an indication representative of said remaining longevity compared with said expected longevity for all of said plurality of electrical activities so that remaining longevity compared with said predetermined expected longevity can be compared among all of said plurality of electrical activities.

12. The medical system of any preceding claim:
wherein said implantable medical device comprises a heart stimulator; and
wherein said plurality of electrical activities comprises at least two of atrial pacing, right ventricular pacing, left ventricular pacing, telemetry and charging.

## Patentansprüche

1. Medizinisches System, mit:
einer implantierbaren medizinischen Vorrichtung (10) mit:
einer Energiequelle (14), die operativ an eine elektrische Schaltung gekoppelt ist und die Schaltung versorgt, wobei die Energiequelle eine Lebensdauer aufweist, **gekennzeichnet durch** eine Entladungstiefe, die repräsentativ für ein erstes Entladestadium, ein zweites Entladestadium und ein drittes Entladestadium ist; und
einer elektrischen Schaltung (12), die operativ an die Energiequelle gekoppelt ist und durch die Energiequelle versorgt wird, wobei die elektrische Schaltung konfiguriert ist, um eine verbleibende Lebensdauer der Energiequelle zu berechnen; und
einem Display (26), das operativ an die elektrische Schaltung gekoppelt ist, das konfiguriert ist, um in graphischer Form unter Verwendung einer Skalenlänge, die repräsentativ für eine Zeit zwischen dem ersten Entladestadium und dem dritten Entladestadium ist, anzuzeigen;
einem ersten Abschnitt des Displays zwischen dem zweiten Entladestadium und dem dritten Entladestadium, die in einer ersten Farbe angezeigt werden;
einem zweiten Abschnitt des Displays zwischen der verbleibenden Lebensdauer und dem zweiten Entladestadium, die in einer zweiten Farbe anzezeigt werden; und
wobei die implantierbare medizinische Vorrichtung (10) in der Lage ist, um konfiguriert zu werden, eine Mehrzahl von elektrischen Aktivitäten abzugeben;
wobei die elektrische Schaltung (12) ferner konfiguriert ist, um eine verbleibende Lebensdauer der Energiequelle separat für jede der Mehrzahl von elektrischen Aktivitäten zu berechnen;
wobei die Energiequelle (14) eine vorbestimmte erwartete Lebensdauer für jede der Mehrzahl von elektrischen Aktivitäten aufweist;
wobei das Display (26) konfiguriert ist, um für jede der Mehrzahl von elektrischen Aktivitäten in graphischer Form einen Hinweis anzuzeigen, der repräsentativ für die verbleibende Lebensdauer verglichen mit der erwarteten Lebensdauer ist.

2. Medizinisches System nach Anspruch 1:
wobei das erste Entladestadium repräsentativ für einen Beginn eines Gebrauchs ist;
wobei das zweite Entladestadium repräsentativ für einen empfohlenen Austauschzeitpunkt ist; und
wobei das dritte Entladestadium repräsentativ für ein Ende eines Gebrauchs ist.

3. Medizinisches System nach Anspruch 1 oder 2, wobei die graphische Form ein Säulendiagramm umfasst.

4. Medizinisches System nach einem der vorstehenden Ansprüche, wobei die Energiequelle eine Batterie umfasst.

5. Medizinisches System nach einem der vorstehenden Ansprüche, wobei die elektrische Schaltung konfiguriert ist, um die verbleibende Lebensdauer unter Verwendung von zumindest einem von einer Spannung der Batterie und einer Zählung einer verbrauchten und abgegebenen Energie durch die implantierbare medizinische Vorrichtung zu berechnen.

6. Medizinisches System nach Anspruch 5, wobei die elektrische Schaltung konfiguriert ist, um die verbleibende Lebensdauer unter Verwendung der Spannung der Batterie zu berechnen.

7. Medizinisces System nach Anspruch 5, wobei die elektrische Schaltung konfiguriert ist, um die verbleibende Lebensdauer unter Verwendung der Zählung einer verbrauchten und abgegebenen Energie durch die implantierbare medizinische Vorrichtung zu berechnen.

8. Medizinisches System nach Anspruch 5, wobei die elektrische Schaltung konfiguriert ist, um die verbleibende Lebensdauer unter Verwendung einer Kombination der Spannung der Batterie und der Zählung einer verbrauchten und abgegebenen Energie durch die implantierbare medizinische Vorrichtung zu berechnen.

9. Medizinisches System nach Anspruch 1:
wobei die graphische Form eine Skalenlänge, die repräsentativ für eine Zeit ist, umfasst, wobei die Skala Indikationen, dass eine Zeit der vorbestimmten erwarteten Lebensdauer genügt, zeigt; und
wobei die verbleibende Lebensdauer separat für jede der Mehrzahl von elektrischen Aktivitäten angezeigt wird, entlang der Skala in Relation zu den Freimachingsvermerken von einer Zeit, die der vorbestimmten erwarteten Lebensdauer genügen.

10. Medizinisches System nach Anspruch 9:
wobei die graphische Form ferner die Skala umfasst, die Indikationen, dass eine Zeit die vorbestimmte erwartete Lebensdauer überschreitet, zeigt.

11. Medizinisches System nach Anspruch 9 oder 10, wobei die graphische Form eine gemeinsame Sicht mit einem Hinweis bereitstellt, der repräsentativ für die verbleibende Lebensdauer verglichen mit der erwarteten Lebensdauer für sämtliche der Mehrzahl von elektrischen Aktivitäten ist, so dass eine verbleibende Lebensdauer verglichen mit der vorbestimmten erwarteten Lebensdauer unter sämtlichen der Mehrzahl von elektrischen Aktivitäten verglichen werden kann.

12. Medizinisches System nach einem der vorstehenden Ansprüche:
wobei die implantierbare medizinische Vorrichtung einen Herzstimulator umfasst;
und wobei die Mehrzahl von elektrischen Aktivitäten zumindest zwei umfasst von atrialer Schrittmachertherapie, rechtsventrikulärer Schrittmachertherapie, linksventrikulärer Schrittmachertherapie, Telemetrie und Aufladung.

## Revendications

1. Système médical, comportant :
un dispositif médical implantable (10) ayant :
une source d'énergie (14) couplée de manière opérationnelle à des circuits électriques et alimentant ceux-ci, ladite source d'énergie ayant une longévité **caractérisée par** une profondeur de décharge représentative d'un premier niveau de décharge, d'un deuxième niveau de décharge et d'un troisième niveau de décharge ; et
des circuits électriques (12) couplés de manière opérationnelle à ladite source d'énergie et étant alimentés par celle-ci, lesdits circuits électriques étant configurés pour calculer une longévité restante de ladite source d'énergie ; et
un affichage (26), couplé de manière opérationnelle auxdits circuits électriques, configuré pour afficher sous une forme graphique en utilisant une longueur d'échelle représentative d'un temps entre ledit premier niveau de décharge et ledit troisième niveau de décharge ;
une première portion dudit affichage entre ledit deuxième niveau de décharge et ledit troisième niveau de décharge étant indiquée dans une première couleur ;
une deuxième portion dudit affichage entre ladite longévité restante et ledit deuxième niveau de décharge étant indiquée dans une deuxième couleur ;
une troisième portion dudit affichage entre ledit premier niveau de décharge et ladite longévité restante étant indiquée dans une troisième couleur ; et
dans lequel ledit dispositif médical implantable (10) peut être configuré pour délivrer une pluralité d'activités électriques ;
dans lequel lesdits circuits électriques (12) sont en outre configurés pour calculer une longévité restante de ladite source d'énergie séparément pour chaque activité de ladite pluralité d'activités électriques ;
dans lequel ladite source d'énergie (14) a une longévité attendue prédéterminée pour chaque activité de ladite pluralité d'activités électriques ;
dans lequel ledit affichage (26) est configuré pour afficher, pour chaque activité de ladite pluralité d'activités électriques, sous une forme graphique, une indication représentative de ladite longévité restante par rapport à ladite longévité attendue.

2. Système médical selon la revendication 1 :
dans lequel ledit premier niveau de décharge est représentatif d'un début de service ;
dans lequel ledit deuxième niveau de décharge est représentatif d'un temps de remplacement recommandé ; et
dans lequel ledit troisième niveau de décharge est représentatif d'une fin de service.

3. Système médical selon la revendication 1 ou 2, dans lequel ladite forme graphique comporte un diagramme en bâtons.

4. Système médical selon l'une quelconque des revendications précédentes, dans lequel ladite source d'énergie comporte une batterie.

5. Système médical selon l'une quelconque des revendications précédentes, dans lequel lesdits circuits électriques sont configurés pour calculer ladite longévité restante en utilisant au moins un élément parmi une tension de ladite batterie et un comptage d'énergie consommée et délivrée par ledit dispositif médical implantable.

6. Système médical selon la revendication 5, dans lequel lesdits circuits électriques sont configurés pour calculer ladite longévité restante en utilisant ladite tension de ladite batterie.

7. Système médical selon la revendication 5, dans lequel lesdits circuits électriques sont configurés pour calculer ladite longévité restante en utilisant ledit comptage d'énergie consommée et délivrée par ledit dispositif médical implantable.

8. Système médical selon la revendication 5, dans lequel lesdits circuits électriques sont configurés pour calculer ladite longévité restante en utilisant une combinaison de ladite tension de ladite batterie et dudit comptage d'énergie consommée et délivrée par ledit dispositif médical implantable.

9. Système médical selon la revendication 1,
dans lequel ladite forme graphique comporte une longueur d'échelle représentative d'un temps, ladite échelle présentant des indices de temps satisfaisant à ladite longévité attendue prédéterminée ; et
dans lequel ladite longévité restante est affichée, séparément pour chaque activité de ladite pluralité d'activités électriques, le long de ladite échelle en lien avec lesdits indices de temps satisfaisant à ladite longévité attendue prédéterminée.

10. Système médical selon la revendication 9 :
dans lequel ladite forme graphique comporte en outre ladite échelle présentant des indices de temps sous ladite longévité attendue prédéterminée et présentant des indices de temps dépassant ladite longévité attendue prédéterminée.

11. Système médical selon la revendication 9 ou 10, dans lequel ladite forme graphique fournit une vue commune avec une indication représentative de ladite longévité restante comparée à ladite longévité attendue pour la totalité de ladite pluralité d'activités électriques de sorte que la longévité restante comparée à ladite longévité attendue prédéterminée peut être comparée parmi toutes les activités de ladite pluralité d'activités électriques.

12. Système médical selon l'une quelconque des revendications précédentes :
dans lequel ledit dispositif médical implantable comporte un stimulateur cardiaque ; et
dans lequel ladite pluralité d'activités électriques comporte au moins deux éléments parmi une stimulation auriculaire, une stimulation ventriculaire droite, une stimulation ventriculaire gauche, une télémesure et une charge.
